# EUROPEAN PATENT APPLICATION

(11) **EP 4 755 438 A1**
(43) Date of publication of application: **10.06.2026**
(21) Application number: 25220479.7
(22) Date of filing: 03.12.2025
(51) Int. Cl.: A61M 60/295, A61M 60/33, A61M 60/13, A61M 60/833, A61M 60/859, A61M 60/861, A61M 60/892, A61M 60/414, A61M 60/221

(54) **RENAL VEIN PULLER PUMP**

(30) Priority: 04.12.2024 US 202463728038 P
(71) Applicant: Abiomed, Inc., Danvers, MA 01923 (US)
(72) Inventor: KYROLLOS, Alfred, Danvers, 01923 (US); CUCHIARA, Michael, Danvers, 01923 (US); UNUDURTHI, Sathya Dev, Danvers, 01923 (US); KIM, Jin Kwang, Danvers, 01923 (US); WESTENFELD, Ralf, Danvers, 01923 (US)
(74) Representative: Wittmer, Maximilian

(57) **Abstract**

A blood pump is provided, that is configured to be disposed within the inferior vena cava (IVC). The blood pump may include a cannula having an inlet and an outlet, and an impeller configured to cause blood to flow through the cannula from the inlet towards the outlet. The inlet may be configured to be disposed at or near where the renal veins connect to the IVC. An occlusive balloon, anchor(s), and/or fixate(s) may be operably coupled to a surface of the cannula. A film may be coupled to anchor(s) and/or fixate(s), to restrict the flow of blood past the film. Restriction of blood flow around the cannula creates a pressure gradient between the inlet and outlet in the IVC, allowing for effective decongestion of the venous compartment distal of the inlet. The anchor(s) and/or fixate(s) may prevent vein collapse during operation due to increased negative pressures.

## Description

### CROSS-REFERENCE TO RELATED APPLICATIONS

This application claims priority to U.S. Provisional Patent Application No. 63/728,038, filed December 4, 2024, the contents of which are incorporated by reference herein in its entirety.

### TECHNICAL FIELD

This disclosure is directed towards blood pumps, and specifically, to blood pumps and techniques for using such pumps for preserving and/or improving renal function.

### BACKGROUND

Renal function is at least partially influenced by cardiac function, and the flow of blood to or from the kidneys. For example, renal congestion occurs when blood flow through the kidneys becomes impaired, leading to a backup of blood and fluid in the kidney's vascular system. This disrupts the normal filtering and drainage processes of the kidneys. Renal congestion may be caused by, *e.g.,* cardiac failure, chronic venous insufficiency, obstructions, and/or blood clots.

### BRIEF SUMMARY

In various aspects, a blood pump configured to be disposed within the inferior vena cava (IVC) may be provided. The blood pump may include a cannula having an inlet and an outlet. The inlet may be configured to be disposed at or near where the renal veins connect to the IVC. The blood pump may include an impeller configured to cause blood to flow through the cannula from the inlet towards the outlet.

In certain aspects, the blood pump may include an occlusion balloon operably coupled to an exterior surface of the cannula. The occlusion ballon may be configured to operate in various states, such as a compressed state and a fully expanded state. The occlusion balloon may be operably coupled to the cannula at or near the inlet and at or near the outlet. The occlusion balloon may be any shape, including, e.g., having a distal conical portion, a proximal conical portion, and an intermediate cylindrical portion between the distal and proximal conical portions. The occlusion balloon may be operably coupled to a lumen passing through at least a portion of the cannula. The occlusion balloon may be composed of any appropriate material, such as a thermoplastic polyurethane (TPU). In certain aspects, the TPU may have a shore hardness of 80A-100A. While various sizes are envisioned, in certain aspects, a maximum outer diameter of the occlusion balloon in the compressed state may be no more than 3 mm, and a maximum outer diameter of the occlusion balloon in the fully expanded state may be 18 mm - 22 mm. In certain aspects, an axial length of the occlusion balloon, may be 42 mm - 52 mm.

In certain aspects, the blood pump may include at least one anchor or fixate operably coupled to a surface (such as an external surface) of the cannula. The anchor(s) or fixate(s) may be located, e.g., at or near the inlet and/or outlet of the cannula. The anchor(s) or fixate(s) may extend axially distally from the inlet, or proximal from the outlet of the cannula. The at least one anchor or fixate may be a single anchor or fixate located at or near the inlet or outlet of the cannula. The at least one anchor or fixate may include a first anchor or fixate at or near the inlet of the cannula and a second anchor or fixate at or near the outlet of the cannula. Each anchor or fixate may be configured to operate in various states, such as a compressed state and an expanded state. Each anchor or fixate may include one or more metal or polymeric strands (such as, *e.g.,* a nitinol strand or wire). Each metal or polymeric strand may be configured to extend radially away from the external surface of the cannula. The anchor(s) or fixate(s) may form or define a concave shape oriented distally (or proximally) in the expanded state. The anchor(s) or fixate(s) may include a flow restricting element, such as a film configured to restrict blood flow past the film when in the expanded state. The film may be, e.g., a polyurethane film. In certain aspects, the film may be configured to prevent *all* blood from flowing around the cannula and past the polyurethane film when in the expanded state. In certain aspects, the film may be configured to prevent only some (but not all) of the blood from flowing around the cannula and past the polyurethane film when in the expanded state. In certain aspects, the film may be only present on anchor(s) or fixate(s) disposed at or near the inlet or outlet. In certain aspects, the film may be present on anchor(s) or fixate(s) disposed at or near both the inlet and outlet.

In various aspects, a system may be provided, that may include a blood pump as disclosed herein, and a controller operably coupled to the blood pump.

In various aspects, a method for preserving or improving renal function may be provided. Such a method may include introducing a blood pump as disclosed herein into the inferior vena cava (IVC) of a patient in an appropriate manner, such as, *e.g.,* through the jugular vein. The inlet of the cannula may be disposed at or near where the renal veins connect to the IVC. The outlet of the cannula may be disposed between the inlet of the cannula and the right atrium. The method may include activating the pump to cause blood to be pulled into the inlet of the cannula and out of the outlet of the cannula. The method may include creating a pressure gradient between the inlet and outlet in the IVC. The pressure gradient may be generated by, e.g., expanding an occlusion balloon operably coupled to an external surface of the cannula, and/or by expanding an anchor or fixate at or near the inlet and/or outlet of the cannula, the anchor or fixate including a film configured to restrict blood flow past the film when the anchor or fixate is in an expanded state. The method may include preventing a vein from collapsing by expanding one or more anchors or fixates to press against an interior surface of the vein. Each anchor or fixate may be disposed at or near the inlet or outlet of the cannula.

### BRIEF DESCRIPTION OF DRAWINGS

The accompanying drawings, which are incorporated in and constitute a part of this specification, illustrate embodiments of the present invention and, together with a general description of the invention given above, and the detailed description of the embodiments given below, serve to explain the principles of the present invention.
Figure 1 is a schematic illustration of a system.
Figure 2 is an illustration of an occlusion balloon in an expanded state.
Figure 3 is an illustration of a cannula with anchors or fixates.
Figure 4 is an illustration of a cannula with anchors or fixates including a flow restriction element.
Figure 5 is an illustration of a cross-sectional side view of a cannula within a blood vessel.
Figures 6A-6F are illustrations of various embodiments of anchors or fixates.
Figures 7A-7C are illustrations of various embodiments of anchors or fixates with flow restriction elements.
Figure 8 is an illustration of a cannula with an occlusion balloon and anchors or fixates.
Figure 9 is a illustration of various embodiments of struts forming a woven mesh.

It should be understood that the appended drawings are not necessarily to scale, presenting a somewhat simplified representation of various features illustrative of the basic principles of the invention. The specific design features of the sequence of operations as disclosed herein, including, for example, specific dimensions, orientations, locations, and shapes of various illustrated components, will be determined in part by the particular intended application and use environment. Certain features of the illustrated embodiments have been enlarged or distorted relative to others to facilitate visualization and clear understanding. In particular, thin features may be thickened, for example, for clarity or illustration.

### DETAILED DESCRIPTION

Disclosed are devices that may be introduced through, e.g., the jugular vein and placed where the inlet is close to where the Renal veins drain into the IVC. The pump may have an occlusion balloon on the cannula that can be inflated. Inflation creates a pressure gradient between the inlet and outlet in the IVC, allowing for effective decongestion of the venous compartment distal of the inlet. This may decongest the kidneys and promote renal function while pump is in use. This may also protect the kidneys from degradation and preserve kidney functions in different patient pathologies. Another configuration that can be combined with the occlusion balloon or without the balloon are anchoring tines around the cannula to prevent collapse of the vein around the pump during operation due to increased negative pressures produced around the inlet. This would also allow for the device to be station in its position and not move once activated.

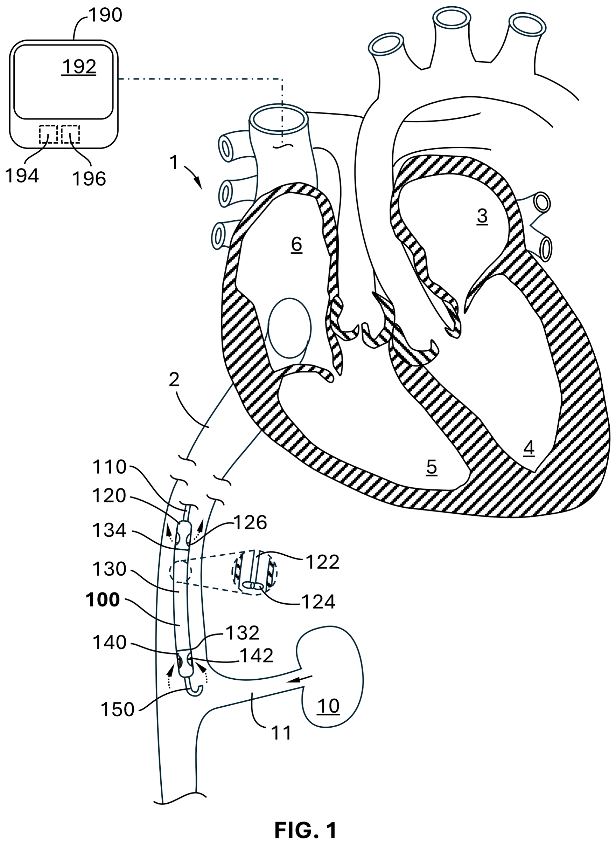

Referring to FIG. 1, the four chambers of a heart (1) are shown - the left atrium (3), left ventricle (4), right ventricle (3), and right atrium (6). A medical device, such as a blood pump (100) introduced through the jugular vein of a patient may enter the heart into the right atrium (6), and then may pass into the inferior vena cava (2), downward, until it reaches the area where the renal vein (11) connects to a kidney (10).

The blood pump (100) may include a catheter (110). A distal end of the catheter may be coupled to a housing (120), which may have a blood flow outlet (126). A distal end of the housing may be coupled to a cannula (130). A distal end of the cannula may have a distal housing (140), which include a blood flow inlet (142). A distal extension (150), which may be a flexible tip, may be coupled to distal end of the blood pump. A rotor or impeller (124) may be coupled to a motor via a drive shaft

(122). The rotor or impeller may be disposed within the cannula (130). When the rotor or impeller rotates, it may cause blood to flow into the blood flow inlet (142), into an inlet (132) of the cannula, through the cannula, out of an outlet (134) of the cannula, and out of the blood pump through the blood flow outlet (126).

The blood pump (100) may be operably coupled to a controller (190). The controller may include a display screen (192), and may include one or more processors (194) operably coupled to non-transitory computer readable storage device(s) (196). The storage device(s) may include instructions that, when executed by the processor(s), causes the processor(s) to control one or more medical devices, such as the blood pump. For example, the controller may be configured to control a rotation speed of the rotor or impeller. The controller may be configured to control the inflation or deflation of an occlusive balloon.

As shown, the blood inlet may be disposed at or near a location where the renal vein connected to the IVC (e.g., typically no more than 25% of the distance along the IVC from where the renal vein connected to the IVC to the right atrium, and preferably no more than 20%, no more than 15%, or no more than 10% of that distance).

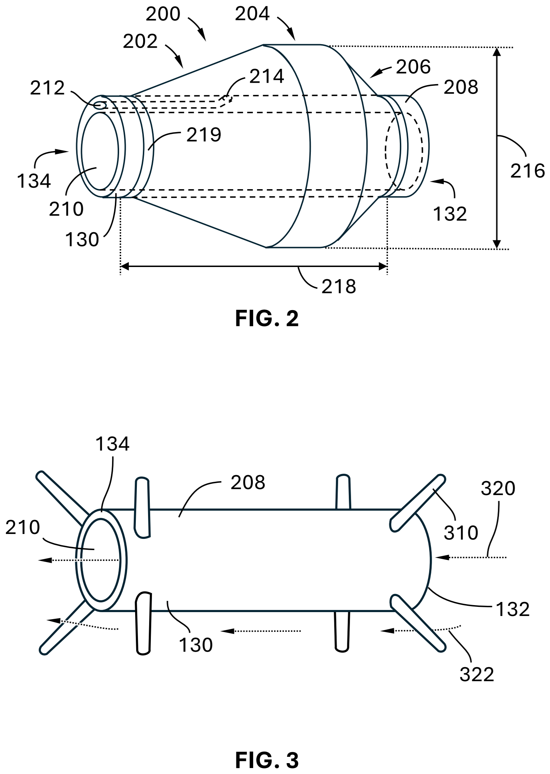

Referring to FIG. 2, an occlusive balloon (200) coupled to a cannula (130) is shown. The cannula (130) is shown as a generally tubular member having a distal end and a proximal end, and a lumen (210) extending from the distal end to the proximal end. The lumen may define the inlet (132) and outlet (134) of the cannula, through which blood may flow. The occlusive balloon (200) may be operably coupled to an external surface (208) of the cannula. In some cases, the occlusion balloon may be operably coupled to the cannula at or near the inlet (e.g., within 25% of the axial length of the cannula from the inlet, and preferably no more than 20%, no more than 15%, or no more than 10% of that distance), and may be coupled at or near the outlet (e.g., within 25% of the axial length of the cannula from the outlet, and preferably no more than 20%, no more than 15%, or no more than 10% of that distance).

The occlusive balloon may be configured to operate in various states, such as a compressed state and a fully expanded state. An example of a fully expanded state is shown in FIG. 2. From a compressed state, a fluid (such as saline) may be introduced into the balloon via an inflation lumen (212) that passes through at least a portion of the cannula, and may exit the cannula into the balloon through a side port (214). As the fluid enters the occlusion balloon, the outer diameter of the occlusion balloon increases, from a starting state that is typically slightly larger than the underlying cannula to which it is attached, to a fully expanded state with the largest diameter. Various sizes are envisioned. in certain aspects, a maximum outer diameter (216) of the occlusion balloon in the compressed state is no more than 3 mm, no more than 2.9 mm, no more than 2.8 mm, or no more than 2.7 mm. In certain aspects, a maximum outer diameter (216) of the occlusion balloon in the fully expanded state may be 15 mm, 16 mm, 17 mm, 18 mm, 19 mm, or 20 mm, up to 20 mm, 21 mm, 22 mm, 23 mm, 24 mm, or 25 mm, including all combinations and subranges thereof.

In addition to the outer diameter, various balloon lengths are also envisioned. The length of the balloon is the maximum axial length (218) of the balloon, including the portions (219) that are coupled to the external surface (208). In certain aspects, an axial length of the occlusion balloon may be 40 mm, 42 mm, 44 mm, or 46 mm up to 46 mm, 48 mm, 50 mm, or 52 mm, including all combinations and subranges thereof.

The shape of the occlusive balloon may vary. For example, the balloon may be generally toroidal or donut-shaped, or generally cylindrical. In some cases, the balloon may have a distal conical portion (206), a proximal conical portion (202), and an intermediate cylindrical portion (204) between the distal and proximal conical portions.

The occlusion balloon may be composed of any appropriate material, such as polyethylene, polyurethane or polyvinyl chloride (PVC). In some cases, the balloon may be composed of a thermoplastic polyurethane (TPU). In certain aspects, the material may have a shore hardness of 80A-100A, such as 80A, 85A, or 90A, up to 90A, 95A, or 100A, including all combinations and subranges thereof.

Referring to FIG. 3, in certain aspects, the blood pump may include at least one anchor or fixate (310) operably coupled to an external surface (208) of the cannula. As used herein, the term "anchor" is used to refer to components that interact with a blood vessel wall via pressure and/or friction only, while "fixate" is used to refer to components that interact with a blood vessel wall with more components that at least partially embed or physically grip the blood walls, such as, e.g., hooks, barbs, etc.

The anchor(s) or fixate(s) may be located at any location along the length of the cannula. In some cases, the anchor(s) or fixate(s) may be located, e.g., at or near the inlet and/or outlet of the cannula.

The total number of anchors or fixates may vary as needed. For example, the at least one anchor or fixate may include a single anchor or fixate located at or near the inlet or outlet of the cannula. In some cases, the anchor(s) or fixate(s) may include a first anchor or fixate at or near the inlet of the cannula and a second anchor or fixate at or near the outlet of the cannula. Each anchor or fixate may be configured to operate in various states, such as a compressed state and an expanded state. For example, an external sheath (not shown) may be used to hold the anchor(s) or fixate(s) in place while the cannula is being introduced to the patient, and when the cannula is positioned correctly, the external sheath may be withdrawn, allowing the anchor(s) and/or fixate(s) to extend to their normal extended positions.

Each anchor or fixate may include one or more metal or polymeric strands (such as, e.g., a nitinol strand or wire). Each metal or polymeric strand may be configured to extend radially away from the external surface of the cannula. Each metal or polymeric strand may extend axially, either distally or proximally. Referring to FIG. 3, it can be seen that the four anchor(s) or fixate(s) (310) at or near the inlet (132) extend radially outward as well as distally, while the four anchor(s) or fixate(s) (310) at or near the outlet (134) extend radially outward as well as proximally. As shown, when in the expanded state, blood may flow through the lumen (210) of the cannula (see fluid path (320)) as well as flow external to the lumen (210), such as around and through the anchor(s) or fixate(s) and past the cannula (see fluid path 322)).

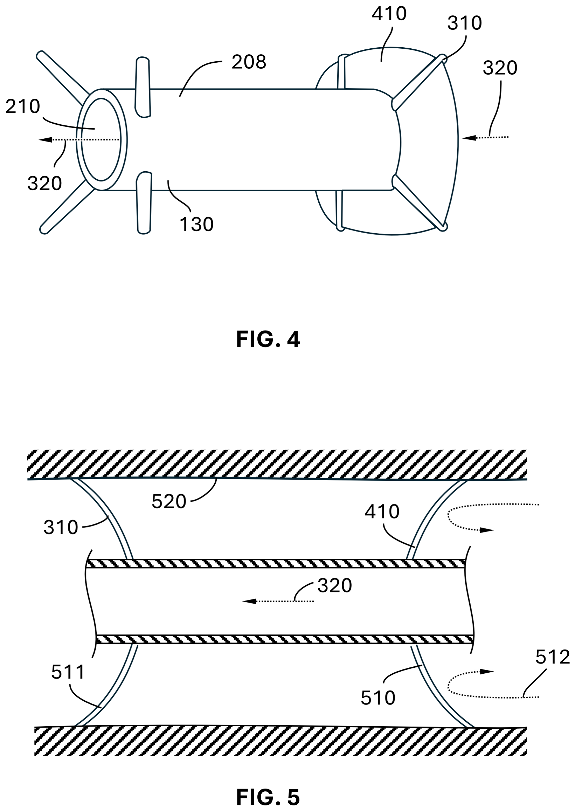

Referring to FIG. 4, the anchor(s) or fixate(s) may include (or may be coupled to) a flow restricting element (410), such as a film configured to restrict blood flow past the film when in the expanded state. The film may be, e.g., polyethylene, polyurethane or polyvinyl chloride (PVC). In some cases, the film may be composed of a thermoplastic polyurethane (TPU). In certain aspects, the film may be configured to prevent *all* blood from flowing around the cannula and past the polyurethane film when in the expanded state, such that the blood may only flow through the lumen (210) (see fluid path (320)). As will be understood, however, in certain aspects, the film may be configured to prevent only some (but not all) of the blood from flowing around the cannula and past the polyurethane film when in the expanded state.

As seen in FIG. 4, in certain aspects, the film (flow restricting element (410)) may only present on anchor(s) or fixate(s) disposed at or near the inlet or outlet. In certain aspects, the film may be present on anchor(s) or fixate(s) disposed at or near both the inlet and outlet.

The anchor(s) or fixate(s) may be disposed in any appropriate shape or form. In some cases, each anchor or fixate is a free-standing anchor or fixate. In some cases, two or more anchors or fixates will intersect, and a crossed pattern of anchors or fixates may be provided. In some embodiments, the anchor(s) or fixate(s) may form a concave shape oriented distally (or proximally) in the expanded state. For example, in FIG. 5, a first set of anchors or fixates (510) form a concave shape oriented distally, while a second set of anchors or fixates (511) form a concave shape oriented proximally. As seen, when a film (flow restricting element (410)) is present to completely block the flow outside of the lumen, blood can flow through the lumen (see fluid path (320)), but fluid otherwise stays upstream of the blood pump due to the film (see fluid path (512)).

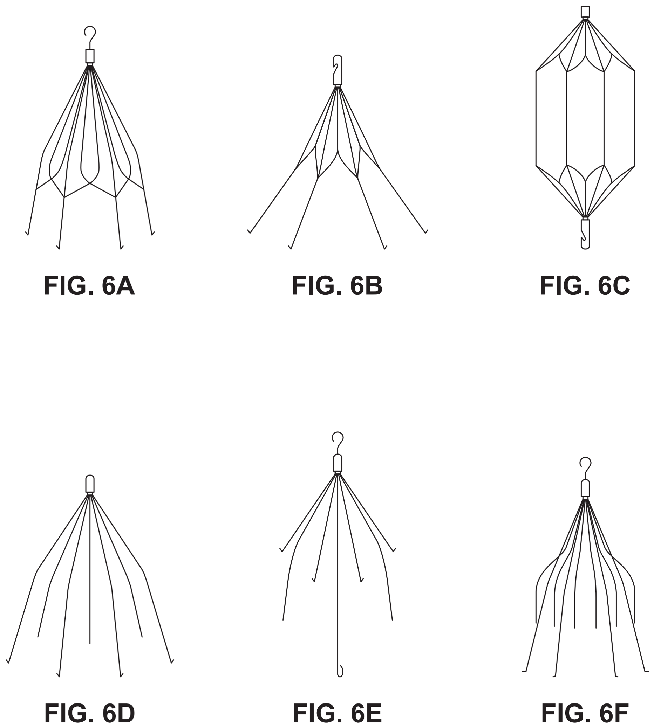

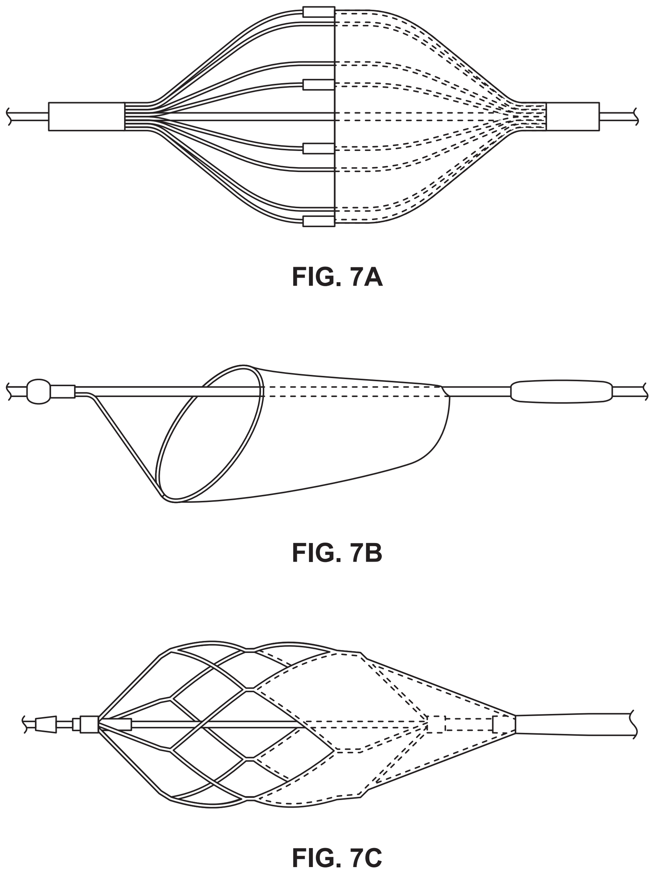

As discussed, various anchor or fixate patterns may be utilized. FIGS. 6A-6F show various configurations of wires or strands used to create various anchor or fixate patterns. FIGS. 7A-7C show various anchors or fixate structures that have been integrated with a flow-restricting film.

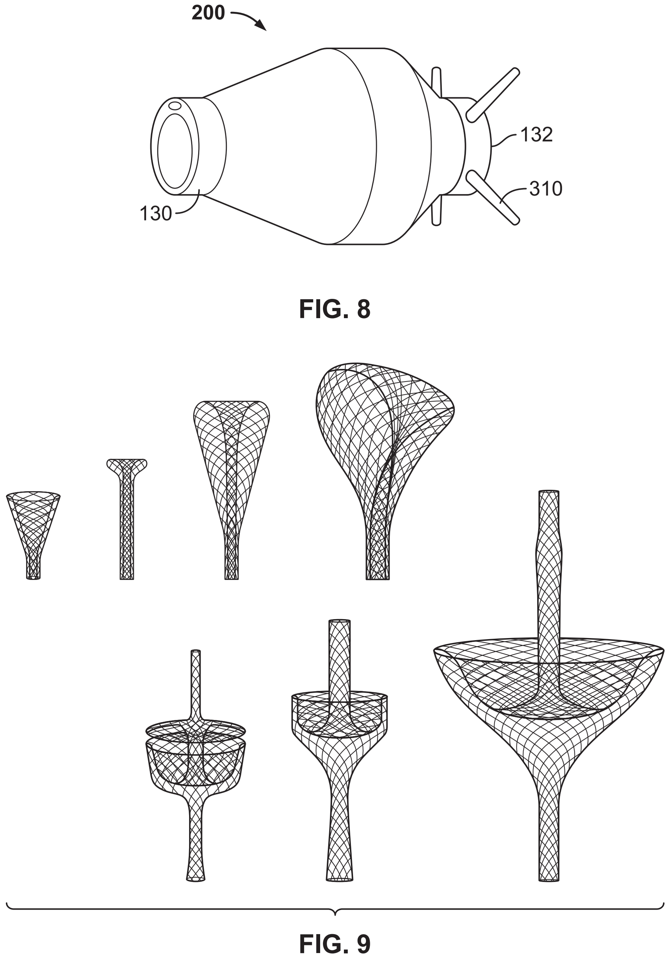

Referring to FIG. 8, it is further envisioned that, in addition to a balloon-only configurations, and anchor/fixate-only configurations, the anchor(s) and/or fixate(s) can also be used in combination with an occlusive balloon. As seen, in some embodiments, the blood pump may include anchor(s) and/or fixate(s) (310) at or near the inlet or outlet, and an occlusive balloon (200) (here, the occlusion balloon is shown as being located proximal to a set of anchor(s) and/or fixate(s) (310) at or near the inlet (132).

Further, referring to FIG. 9, it is also envisioned that several struts may be woven together to form a woven mesh. Such a mesh can be operably coupled to the cannula. In some cases, the mesh may be configured to extend axially in a direction distal from the inlet to the cannula, or extend axially in a direction proximal from the outlet to the cannula.

In various aspects, a method for preserving or improving renal function may be provided. Such a method may include introducing a blood pump as disclosed herein into the inferior vena cava (IVC) of a patient in an appropriate manner, such as, *e.g.,* through the jugular vein. The inlet of the cannula may be disposed at or near where the renal veins connect to the IVC. The outlet of the cannula may be disposed between the inlet of the cannula and the right atrium - that is, as shown in FIG. 1, the inlet (132) may be further from the right atrium (6) than the outlet (134). The method may include activating the pump (e.g., causing the rotor or impeller (124) to rotate), thereby causing blood to be pulled into the inlet (132) of the cannula, through a lumen (210) of the cannula, and out of the outlet (134) of the cannula.

The method may include creating a pressure gradient between the inlet and outlet in the IVC. The pressure gradient may be generated by, e.g., expanding an occlusion balloon operably coupled to an external surface of the cannula, and/or by expanding an anchor or fixate at or near the inlet and/or outlet of the cannula, the anchor or fixate including a film configured to restrict blood flow past the film when the anchor or fixate is in an expanded state. The method may include preventing a vein from collapsing by expanding one or more anchors or fixates to press against an interior surface of the vein. Each anchor or fixate may be disposed at or near the inlet or outlet of the cannula. To move the blood pump, the occlusive balloon (if used) may be placed in a compressed state, the anchor(s) and/or fixate(s) (if used) may be placed in a compressed state, and the blood pump may then be moved as desired.

Various modifications may be made to the systems, methods, apparatus, mechanisms, techniques, and portions thereof described herein with respect to the various figures, such modifications being contemplated as being within the scope of the invention. For example, while a specific order of steps or arrangement of functional elements is presented in the various embodiments described herein, various other orders/arrangements of steps or functional elements may be utilized within the context of the various embodiments. Further, while modifications to embodiments may be discussed individually, various embodiments may use multiple modifications contemporaneously or in sequence, compound modifications and the like.

Although various embodiments which incorporate the teachings of the present invention have been shown and described in detail herein, those skilled in the art can readily devise many other varied embodiments that still incorporate these teachings. Thus, while the foregoing is directed to various embodiments of the present invention, other and further embodiments of the invention may be devised without departing from the basic scope thereof.

## Claims

1. A blood pump configured to be disposed within an inferior vena cava (IVC), comprising:
a cannula having an inlet and an outlet, the inlet configured to be disposed at or near where a renal vein connects to the IVC; and
an impeller configured to cause blood to flow through the cannula from the inlet towards the outlet.

2. The blood pump of claim 1, further comprising an occlusion balloon operably coupled to a surface of the cannula, the occlusion balloon having at least a compressed state and a fully expanded state.

3. The blood pump of claim 2, wherein the occlusion balloon is operably coupled to the cannula at or near the inlet and at or near the outlet;
wherein the occlusion balloon has a distal conical portion, a proximal conical portion, and an intermediate cylindrical portion between the distal conical portion and the proximal conical portion; wherein the occlusion balloon is operably coupled to a lumen passing through at least a portion of the cannula; and/or
wherein the occlusion balloon is composed of a thermoplastic polyurethane (TPU).

4. The blood pump of claim 2 or 3, wherein a maximum outer diameter of the occlusion balloon in the compressed state is no more than 3 mm, and a maximum outer diameter of the occlusion balloon in the fully expanded state is 18 mm - 22 mm; and/or
wherein an axial length of the occlusion balloon, is 42 mm - 52 mm.

5. The blood pump of any one of claim 1-4, further comprising at least one anchor or fixate operably coupled to a surface of the cannula at or near the inlet and/or outlet of the cannula.

6. The blood pump of claim 5, wherein the at least one anchor or fixate is a single anchor or fixate at or near the inlet or outlet of the cannula; or
wherein the at least one anchor or fixate is a first anchor or fixate at or near the inlet of the cannula and a second anchor or fixate at or near the outlet of the cannula.

7. The blood pump of claim 5 or 6, wherein each anchor or fixate is configured to have at least a compressed state and an expanded state.

8. The blood pump of claim 7, wherein each anchor or fixate includes one or more metal or polymeric strands, each metal or polymeric strand configured to extend radially away from the surface of the cannula;
wherein at least one anchor or fixate forms a concave shape oriented distally in the expanded state; and/or
wherein at least one anchor or fixate forms a concave shape oriented proximally in the expanded state.

9. The blood pump of claim 7 or 8, wherein one or more anchors or fixates includes a polyurethane film configured to restrict blood flow past the polyurethane film when in the expanded state.

10. The blood pump of claim 9, wherein the polyurethane film is configured to prevent all blood from flowing around the cannula and past the polyurethane film when in the expanded state;
wherein the polyurethane film is configured to prevent only some blood from flowing around the cannula and past the polyurethane film when in the expanded state;
wherein only one anchor or fixate includes the polyurethane film; or
wherein two anchors or fixates includes the polyurethane film.

11. The blood pump of any one of claims 1-10, further comprising a plurality of struts configured to form a woven mesh operably coupled to a surface of the cannula.

12. A system, comprising:
a blood pump of any one of claims 1-11; and
a controller operably coupled to the blood pump.

13. A method for preserving or improving renal function, comprising:
introducing a blood pump of any one of claims 1-11 into the inferior vena cava (IVC) of a patient, the inlet of the cannula being disposed at or near where a renal vein connects to the IVC, the outlet of the cannula being disposed between the inlet of the cannula and a right atrium; and
activating the blood pump to cause blood to be pulled into the inlet of the cannula and out of the outlet of the cannula.

14. The method of claim 13, further comprising creating a pressure gradient between the inlet and outlet in the IVC;
further comprising preventing a vein from collapsing by expanding one or more anchors or fixates to press against an interior surface of the vein, each anchor or fixate disposed at or near the inlet or outlet of the cannula; or
wherein the blood pump is introduced through a jugular vein.

15. The method of claim 14, wherein the pressure gradient is generated by expanding an occlusion balloon operably coupled to a surface of the cannula; or
wherein the pressure gradient is generated by expanding an anchor or fixate at or near the inlet and/or outlet of the cannula, the anchor or fixate including a polyurethane film configured to restrict blood flow past the polyurethane film when the anchor or fixate is in an expanded state.
